# EUROPEAN PATENT SPECIFICATION

(11) **EP 3 658 177 B1**
(45) Date of publication and mention of the grant of the patent: **20.03.2024**
(21) Application number: 18752615.7
(22) Date of filing: 27.07.2018
(51) Int. Cl.: A61K 38/48, A61K 38/16, A61P 21/00, G06G 1/00

(54) **TOOL FOR DOSING BOTULINUM NEUROTOXIN**
HILFSMITTEL ZUR DOSIERUNG VON BOTULINUM-NEUROTOXIN
OUTIL DE DOSAGE DE LA NEUROTOXINE BOTULIQUE

(30) Priority: 27.07.2017 US 201762537567 P
(43) Date of publication of application: 03.06.2020
(73) Proprietor: Ipsen Biopharm Limited, Wrexham LL13 9UF (GB)
(72) Inventor: PEARL, Donald, Basking Ridge New Jersey 07920 (US); SANTOS, Ayanna, Basking Ridge New Jersey 07920 (US)
(74) Representative: Hobson, David James
(86) International application number: PCT/GB2018/052149
(87) International publication number: WO 2019/021024

(56) References cited:
- WO-A1-2008/131941
- CN-U- 201 673 426
- DE-A1- 2 428 058
- DE-U1- 29 814 563
- GB-A- 734 595
- GB-A- 1 476 668
- US-A1- 2001 050 242
- US-A1- 2010 124 559
- E. C DAVIS: "Botulinum toxin and spasticity", JOURNAL OF NEUROLOGY NEUROSURGERY & PSYCHIATRY., vol. 69, no. 2, 1 August 2000 (2000-08-01) , pages 143-147, XP055512316, GB ISSN: 0022-3050, DOI: 10.1136/jnnp.69.2.143
- Carlos Henrique F. Camargo ET AL: "Botulinum toxin type A in the treatment of lower-limb spasticity in children with cerebral palsy", Arquivos de Neuro-Psiquiatria, 1 March 2009 (2009-03-01), pages 62-68, XP055512321, Retrieved from the Internet: URL:http://www.scielo.br/pdf/anp/v67n1/a16 v67n1.pdf
- Daniela Semedo: "FDA Approves Dysport for Lower Limb Spasticity in Pediatric Patients", , 8 August 2016 (2016-08-08), XP002785328, Retrieved from the Internet: URL:https://cerebralpalsynewstoday.com/201 6/08/08/ipsen-biopharmaceuticals-fda-appro ves-dysport-pediatric-lower-limb-spasticit y/ [retrieved on 2018-10-04]
- DATABASE MEDLINE [Online] US NATIONAL LIBRARY OF MEDICINE (NLM), BETHESDA, MD, US; October 2002 (2002-10), DURSUN N ET AL: "The role of botulinum toxin a in the management of lower limb spasticity in patients with cerebral palsy.", XP002785329, Database accession no. NLM12425363
- TEIVE H ET AL: "P1.071 Botulinum toxin type A in the treatment of lower-limb spasticity in children with cerebral palsy", PARKINSONISM AND RELATED DISORDERS, ELSEVIER SCIENCE, OXFORD, GB, vol. 15, 1 December 2009 (2009-12-01), page S47, XP026805633, ISSN: 1353-8020 [retrieved on 2009-12-01]
- MARISSA BARLAAN LUKBAN ET AL: "Effectiveness of botulinum toxin A for upper and lower limb spasticity in children with cerebral palsy: a summary of evidence", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 116, no. 3, 14 January 2009 (2009-01-14), pages 319-331, XP019722606, ISSN: 1435-1463

## Description

The invention relates to a tool as claimed for use in determining the dosage amount of an active agent to be administered to a subject.

### BACKGROUND OF THE INVENTION

Spasticity is a condition in which there is an abnormal increase in muscle tone or stiffness in one or more muscles, which might interfere with movement thereof. Spasticity is typically caused by damage to nerve pathways in the brain or spinal cord that control muscle movement, and may occur in association with cerebral palsy, spinal cord injury, multiple sclerosis, stroke, and brain or head trauma.

Lower limb spasticity commonly involves spasticity in the gastrocnemius and soleus muscle complex located in the calf. These calf muscles are the chief extensors of the foot at the ankle-joint. In walking, they work to raise the heel from the ground.

Symptoms of spasticity may include increased muscle tone, rapid muscle contractions, exaggerated deep tendon reflexes, and/or muscle spasms. The degree of spasticity can vary from mild muscle stiffness to severe, painful, and uncontrollable muscle spasms.

It is known to treat spasticity in adults using botulinum neurotoxin. Applicant has found that botulinum neurotoxin may be used to treat lower limb spasticity in subjects 18 years of age or under, for example subjects between 2 and 17 years of age.

Botulinum neurotoxin is produced by *Clostridium botulinum* in the form of a large protein complex, consisting of botulinum neurotoxin itself complexed to a number of accessory proteins. There are at present seven different classes of botulinum neurotoxin, namely: botulinum neurotoxin serotypes A, B, C, D, E, F and G, all of which share similar structures and modes of action. Different BoNT serotypes can be distinguished based on inactivation by specific neutralizing anti-sera, with such classification by serotype correlating with percentage sequence identity at the amino acid level. Botulinum neurotoxin proteins of a given serotype are further divided into different subtypes on the basis of amino acid percentage sequence identity.

Botulinum toxin type A is mainly commercially available from Ipsen (Dysport^{®}, Ipsen Limited, Slough, UK) and Allergan (BOTOX^{®}, Allergan Inc., Irvine, CA, USA) whereas botulinum toxin type B is sold by Elan (Myobloc^{®}/Neurobloc^{®}, Solstice Neurosciences Inc., San Diego, CA, USA).

US 2001/050242 A1 describes a weight-specific elixir dosage calculator. GB 1476668 A describes a device for calculating drug doses. DE 29814563 U1 describes a device for the patient-dependent determination of a dosage of a medicament. DE 2428058 A1 describes the calculation of a pharmacokinetic variable using tables and slide rules with scales of dosage values relating to drug doses. GB 734595 A describes a medicinal dose computer. CN 201673426 U describes a device for calculating paediatric medicament doses.

### SUMMARY OF THE INVENTION

The present invention is defined by the claims. The tool as claimed may be used in a method of treating lower limb spasticity in a subject, particularly one which is 18 years of age or under, for example between 2 and 17 years of age, comprising administering to the subject an effective amount of botulinum neurotoxin.

In an embodiment, the botulinum neurotoxin is of types A, B, C, D, E, F, and/or G.

In a particular embodiment, the botulinum neurotoxin is of type A.

In use, the botulinum neurotoxin may be administered unilaterally. In use, botulinum neurotoxin may be administered to an individual leg in an amount of 10 to 15 units per kilogram of the total body weight of the subject.

In use, the botulinum neurotoxin may be administered bilaterally. In use, each leg of the subject may be individually administered with 10 to 15 units per kilogram of the total body weight of the subject.

In use, the total amount of botulinum neurotoxin administered to the subject per treatment session may be 1000 units or less.

In use, botulinum neurotoxin may be administered at more than one site in any single muscle.

In some uses, no more than 0.5 mL of Dysport^{®} may be administered at any single site.

In some uses, a subsequent treatment session may not take place within 12 weeks of another treatment session. In use, a subsequent treatment session may take place 16 to 22 weeks after another treatment session.

In use, botulinum neurotoxin may be administered to a subject in an amount of at least about 5 units per kilogram of the total body weight of the subject. Botulinum neurotoxin may be administered to a subject in an amount of at least about 10 units per kilogram of the total body weight of the subject. The amount of botulinum neurotoxin administered to a subject may be less than about 15 units per kilogram of the total body weight of the subject (e.g. less than about 12 units per kilogram of the total body weight of the subject). The values disclosed in this paragraph may relate to the number of units administered per leg of a subject.

In use, the botulinum neurotoxin may be administered to the gastrocnemius-soleus complex.

In use, botulinum neurotoxin may be administered unilaterally in an amount of 10 to 15 units per kilogram of the total body weight of the subject, preferably wherein said botulinum neurotoxin is administered to a gastrocnemius-soleus complex of the subject.

In use, botulinum neurotoxin may be administered bilaterally in an amount of 20 to 30 units per kilogram of the total body weight of the subject, preferably wherein said botulinum neurotoxin is administered to a gastrocnemius-soleus complex of the subject.

In use, the botulinum neurotoxin may be administered to a gastrocnemius of the subject. In use, botulinum neurotoxin may be administered to an individual gastrocnemius in an amount of 6 to 9 units per kilogram of the total body weight of the subject.

In use, each gastrocnemius of the subject may be administered with such an amount.

In use, the botulinum neurotoxin may be administered to a soleus of the subject.

In use, botulinum neurotoxin may be administered to an individual soleus in an amount of 4 to 6 units per kilogram of the total body weight of the subject.

In use, each soleus of the subject may be administered with such an amount.

In use, the botulinum neurotoxin may be administered by injection to a muscle.

In use, up to 4 injections may be made to a gastrocnemius.

In use, up to 2 injections may be made to a soleus. In use, injection sites may be determined by palpation or by the use of injection guiding techniques such as electromyography or electrical stimulation.

In use, botulinum neurotoxin may be administered in a dosage amount determined based in part on the weight of the subject, whether administration is to be unilateral or bilateral, whether administration will be to the gastrocnemius and/or the soleus, the severity of the spasticity, what muscles are affected by the spasticity, the presence of local muscle weakness, and/or the subject's history with treatment with botulinum neurotoxin.

In use, botulinum neurotoxin may be administered as part of a method comprising the steps of:
(A) obtaining the weight of the subject; (B) choosing unilateral or bilateral administration; (C) based on the weight of the patient and whether administration is to be unilateral or bilateral, determining the amount of botulinum neurotoxin to administer to the subject; and (D) administering botulinum neurotoxin in such an amount to a subject in need of such treatment.

The invention relates to a tool for use in determining a dosage amount of a botulinum neurotoxin to be administered to
a subject, the tool comprising:
   a first member having thereon a first row of numbers indicative of the weight of the subject and a second row of numbers indicative of a dosage amount of the botulinum neurotoxin to be administered to the subject, wherein the numbers in the first row indicating a specific weight are in latitudinal or radial alignment with corresponding numbers in the second row indicating the specific dosage amount to be administered to a subject having that specific weight, wherein the dosage amount corresponds to 5 to 15 units per kilogram of the total body weight of the subject per leg; and
   a second member;
wherein the first and second members are in a relationship with one another such that one member may be moved by a user to provide an indication as to the dosage amount of the botulinum neurotoxin to be administered to a subject having a particular weight; and
   wherein the first member further has thereon a row of numbers indicative of the dosage amount of the botulinum neurotoxin to be administered to a particular muscle of the subject and the numbers in the row which indicate the specific dosage amount to be administered to the muscle of a subject having a specific weight are in longitudinal or radial alignment with corresponding numbers in the first row indicating that specific weight, wherein the dosage amount corresponds to 6 to 9 units per kilogram of the total body weight of the subject to be administered to an individual gastrocnemius muscle of the subject or wherein the dosage amount corresponds to 4 to 6 units per kilogram of the total body weight of the subject to be administered to an individual soleus muscle of the subject.

In an embodiment, both first and second members are planar surfaces that are in a longitudinal sliding arrangement with each other, with the second member overlaying the first member and having an opening or openings that allow(s) for the rows on the first member to be viewed.

In another embodiment, the first member is a planar surface and the second member is a cursor in a longitudinal sliding arrangement with respect to the first member.

In another embodiment, the first member is a circular planar surface and the second member is a dial that pivots around the center point of the first member.

In use, a botulinum neurotoxin may be part of a composition comprising an effective amount of botulinum neurotoxin.

The composition may comprise 10 to 15 units botulinum neurotoxin per kilogram of the total body weight of the subject for each leg to which botulinum neurotoxin is to be administered.

The composition may comprise an amount of botulinum neurotoxin to be administered to an individual gastrocnemius of the subject, the amount being 6 to 9 units per kilogram of the total body weight of the subject.

The composition may comprise an amount of botulinum neurotoxin to be administered to an individual soleus of the subject, the amount being 4 to 6 units per kilogram of the total body weight of the subject.

The composition may be in the form of a lyophilized powder.

The composition may be formed by reconstituting lyophilized powder comprising botulinum neurotoxin in a sodium chloride solution. The composition may comprise 300 to 500 units of botulinum neurotoxin.

The composition may comprise botulinum neurotoxin and human serum albumin.

### DESCRIPTION OF THE DRAWINGS

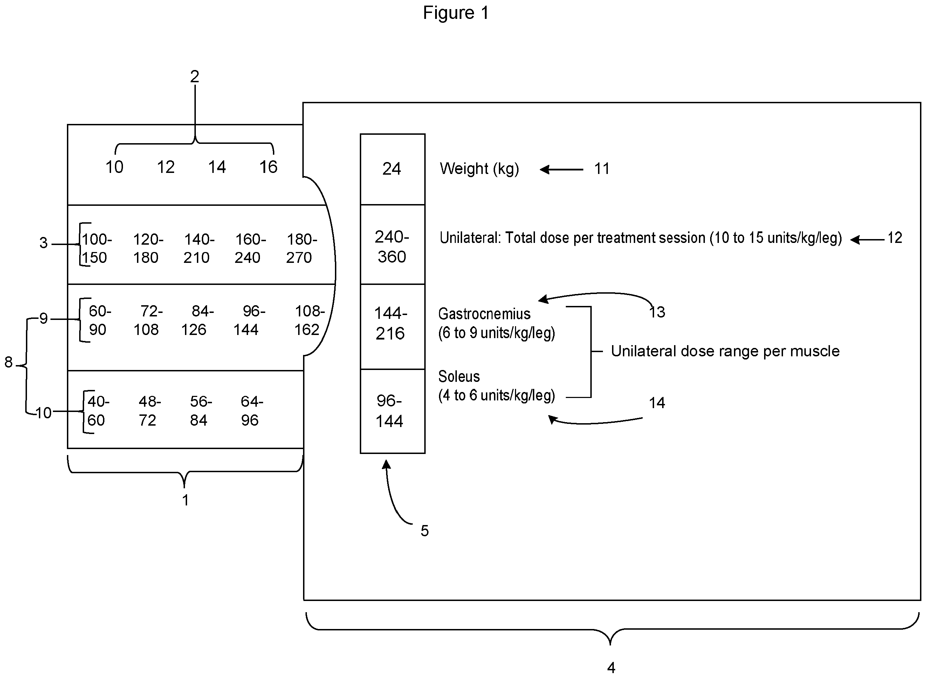

**Figure 1** depicts an embodiment of the tool of the present invention. The tool comprises: (A) a first member (1) having thereon a first row of numbers (2) indicative of the weight of the subject, a second row of numbers (3) indicative of a dosage amount of the active agent to be administered to the subject having a specific weight, and additional rows of numbers (8) with one row indicating the dosage amount to be administered to the gastrocnemius of the subject's leg (9) and another row indicating the dosage amount to be administered to the soleus of the subject's leg (10); and (B) a second member (4). Both first and second members are planar surfaces and are in a relationship with one another such that one member may be moved by a user to provide an indication as to the dosage amount of the active agent to be administered to a subject having a particular weight. The second member overlays the first and contains an opening (5) that allows for the rows on the first member to be viewed. A first marking (11) on the second member is aligned with the row of numbers which indicate the weight of the subject (2), a second marking (12) on the second member is aligned with the row of numbers which indicate the dosage amount of the active agent to be administered to the subject (3), and third (13) and fourth markings (14) on the second member are aligned respectively with the row indicating the dosage amount of the active agent to be administered to the gastrocnemius of the subject's leg (9) and the row indicating the dosage amount of the active agent to be administered to the soleus of the subject's leg (10).

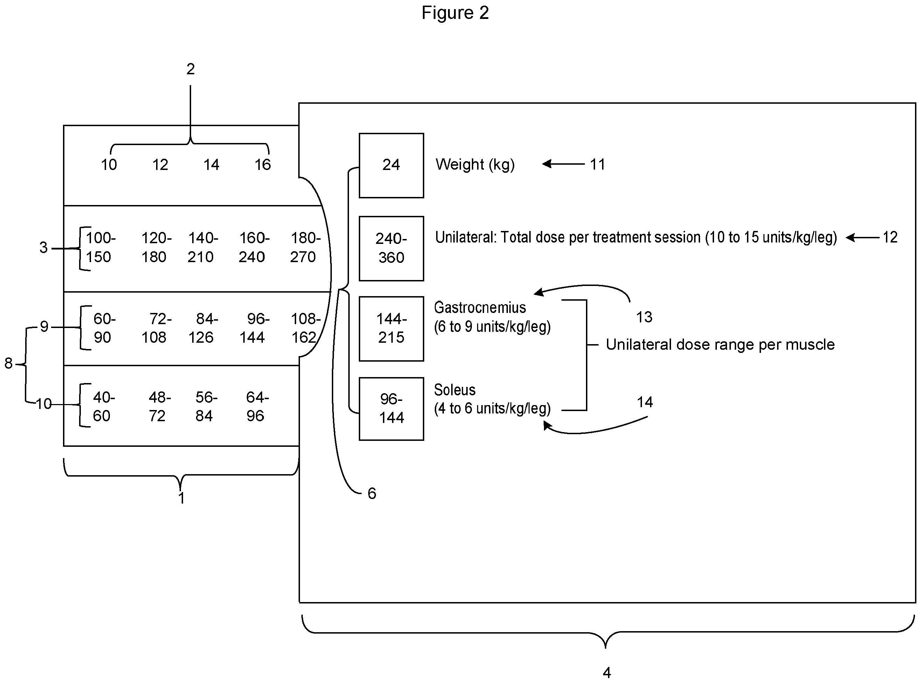

**Figure 2** depicts another embodiment of the tool of the present invention. The tool comprises: (A) a first member (1) having thereon a first row of numbers (2) indicative of the weight of the subject, a second row of numbers (3) indicative of a dosage amount of the active agent to be administered to the subject having a specific weight, and additional rows of numbers (8) with one row indicating the dosage amount to be administered to the gastrocnemius of the subject's leg (9) and another row indicating the dosage amount to be administered to the soleus of the subject's leg (10); and (B) a second member (4). Both first and second members are planar surfaces and are in a relationship with one another such that one member may be moved by a user to provide an indication as to the dosage amount of the active agent to be administered to a subject having a particular weight. The second member overlays the first and contains an openings (6) which each allow for one row on the first member to be viewed. A first marking (11) on the second member is aligned with the row of numbers which indicate the weight of the subject (2), a second marking (12) on the second member is aligned with the row of numbers which indicate the dosage amount of the active agent to be administered to the subject (3), and third (13) and fourth markings (14) on the second member are aligned respectively with the row indicating the dosage amount of the active agent to be administered to the gastrocnemius of the subject's leg (9) and the row indicating the dosage amount of the active agent to be administered to the soleus of the subject's leg (10).

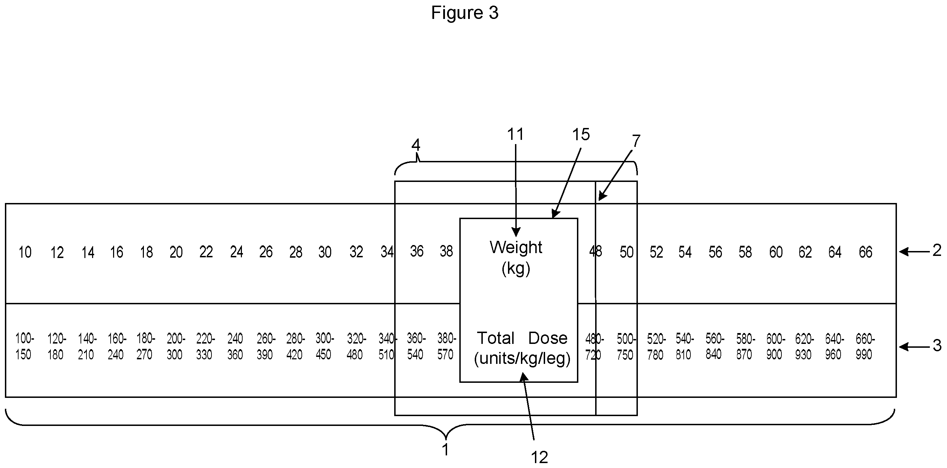

**Figure 3** The tool comprises: (A) a first member (1) having thereon a first row of numbers (2) indicative of the weight of the subject and a second row of numbers (3) indicative of a dosage amount of the active agent to be administered to the subject having a specific weight; and (B) a second member (4). The first member is a planar surface and the second member is a cursor. The second member is in a longitudinal sliding relationship with the first member such that it may be by a user to provide an indication as to the dosage amount of the active agent to be administered to a subject having a particular weight. The cursor is made of transparent material. A first marking (11) on the second member is aligned with the row of numbers which indicate the weight of the subject (2) and a second marking (12) on the second member is aligned with the row of numbers which indicate the dosage amount of the active agent to be administered to the subject (3). The numbers appear on a label (15). A line (7) is present on the cursor to provide an indication of the specific weight and dosage amount to be administered to a subject having that specific weight.

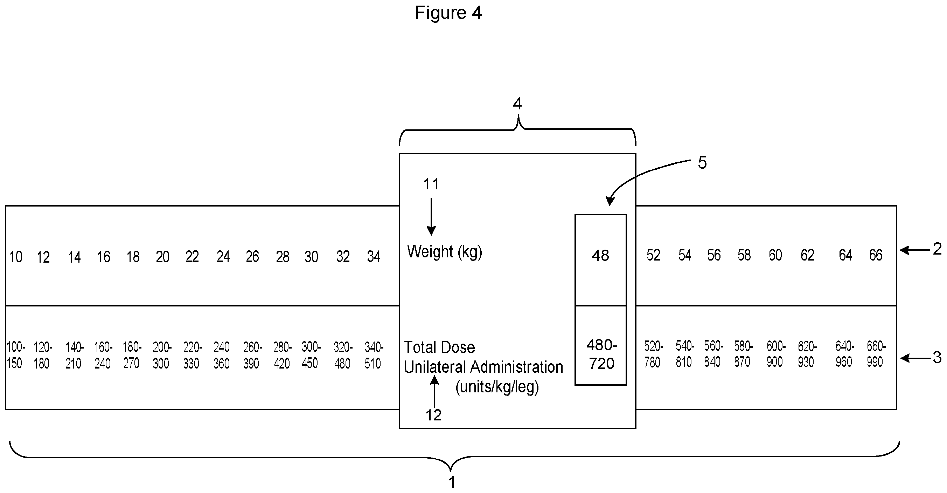

**Figure 4** The tool comprises: (A) a first member (1) having thereon a first row of numbers (2) indicative of the weight of the subject and a second row of numbers (3) indicative of a dosage amount of the active agent to be administered to the subject having a specific weight; and (B) a second member (4). The first member is a planar surface and the second member is a cursor. The second member is in a longitudinal sliding relationship with the first member such that it may be by a user to provide an indication as to the dosage amount of the active agent to be administered to a subject having a particular weight. A first marking (11) on the second member is aligned with the row of numbers which indicate the weight of the subject (2) and a second marking (12) on the second member is aligned with the row of numbers which indicate the dosage amount of the active agent to be administered to the subject (3). The numbers appear on a label (15). The cursor contains an opening (5) that allows the user to view a specific weight amount and dosage amount to be administered to a subject having that specific weight.

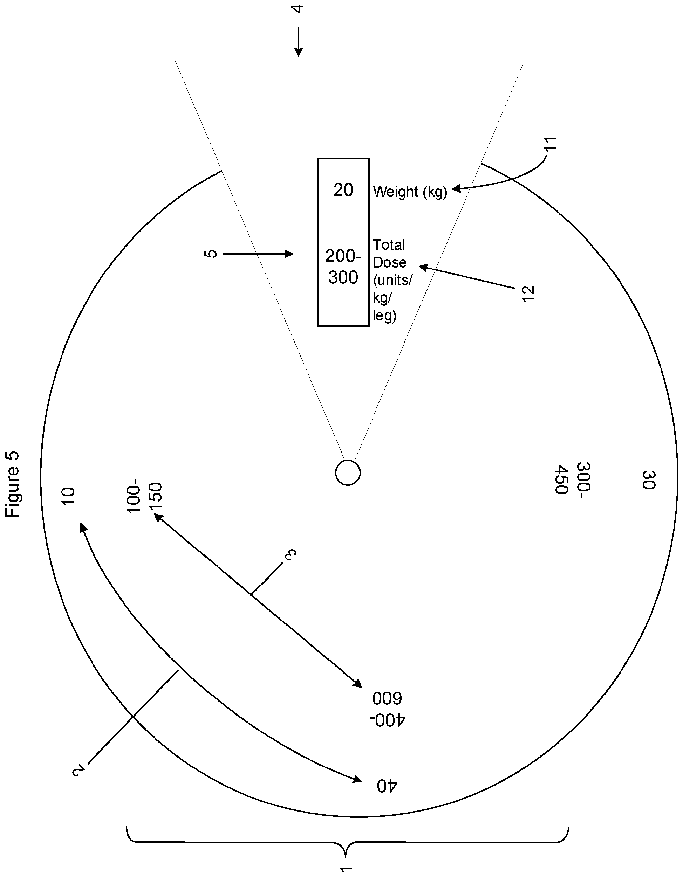

**Figure 5** The tool comprises: (A) a first member (1) having thereon a first row of numbers (2) indicative of the weight of the subject and a second row of numbers (3) indicative of a dosage amount of the active agent to be administered to the subject having a specific weight; and (B) a second member (4). The first member is a circular planar surface and the second member is a dial that pivots around the center point of the first member such that it may be by a user to provide an indication as to the dosage amount of the active agent to be administered to a subject having a particular weight. The cursor contains an opening (5) that allows the user to view a specific weight amount and dosage amount to be administered to a subject having that specific weight. A first marking (11) on the second member is aligned with the row of numbers which indicate the weight of the subject (2) and a second marking (12) on the second member is aligned with the row of numbers which indicate the dosage amount of the active agent to be administered to the subject (3).

### DETAILED DESCRIPTION OF THE INVENTION

In describing the invention, where a range of values is provided with respect to an embodiment, it is understood that each intervening value is encompassed within the embodiment.

A "subject" as used herein refers to a mammal, in particular a human, in need of treatment for lower limb spasticity. For example, a subject may be a human patient in need of such treatment.

Botulinum neurotoxins are proteins produced by *Clostridium botulinum.* These proteins serve as neurotoxins. Upon selective uptake by endocytosis at nerve terminals, they block the release of synaptic vesicles. There are seven known serotypes of botulinum neurotoxin, specifically serotypes A, B, C, D, E, F, and G. As used herein, "botulinum neurotoxin" refers to naturally occurring botulinum neurotoxins, including in their high purity form, and recombinantly engineered botulinum neurotoxins, including those that contain modifications as compared to wild-type botulinum neurotoxins (*e.g*. in their amino acid sequence) so as to have similar or better properties than the wild-type botulinum neurotoxins. Botulinum toxin type A includes all types of botulinum toxin type A, including A1, A2 and A3. Botulinum toxin type C includes all types of botulinum toxin type C, including C1 or C2. The same applies mutatis mutandis to the other serotypes of toxins.

A "high purity botulinum neurotoxin" (whether of type A, B, C, D, E, F or G) refers to botulinum neurotoxin that is free from complexes with other proteins.

In an embodiment, the botulinum neurotoxin is of types A, B, C, D, E, F, and/or G or a recombinantly engineered botulinum neurotoxin having an amino acid sequence that has at least 50%, 60%, 70%, 80%, 90%, 95%, 97%, 98%, or 99% sequence identity (preferably at least 90% sequence identity) to the amino acid sequence of a botulinum neurotoxin of types A, B, C, D, E, F, or G.

Exemplary amino acid sequences for botulinum neurotoxin of types A, B, C, D, E, F, and G are presented as SEQ ID NOs: 1-7, respectively.

The "percent sequence identity" between two or more nucleic acid or amino acid sequences is a function of the number of identical positions shared by the sequences. Thus, % identity may be calculated as the number of identical nucleotides / amino acids divided by the total number of nucleotides / amino acids, multiplied by 100. Calculations of % sequence identity may also take into account the number of gaps, and the length of each gap that needs to be introduced to optimize alignment of two or more sequences. Sequence comparisons and the determination of percent identity between two or more sequences can be carried out using specific mathematical algorithms, such as BLAST, which will be familiar to a skilled person.

In an embodiment, the botulinum neurotoxin is of type A, also known as botulinum neurotoxin A. Botulinum neurotoxin A is sold as Dysport^{®} by Ipsen Biopharmaceuticals, Inc. (Basking Ridge, New Jersey) and as Botox^{®} by Allergan, Inc. (Parsippany-Troy Hills, New Jersey).

In use, the botulinum neurotoxin may be administered unilaterally. The term "unilateral," and its variants, when used herein in reference to administration refers to the administration of botulinum neurotoxin to one leg of the subject.

Thus, in use, botulinum neurotoxin may be administered to a subject unilaterally in an amount of at least about 5 units per kilogram of the total body weight of the subject. In use, botulinum neurotoxin may be administered to a subject unilaterally in an amount of at least about 10 units per kilogram of the total body weight of the subject. The amount of botulinum neurotoxin administered to a subject unilaterally may be less than about 15 units per kilogram of the total body weight of the subject (e.g. less than about 12 units per kilogram of the total body weight of the subject).

In use, botulinum neurotoxin may be administered unilaterally in an amount of 10 to 15 units per kilogram of the total body weight of the subject. As used herein, the term "unit" refers to a unit dose of Dysport^{®}, which refers to the median intraperitoneal LD₅₀ dose of Dysport^{®} in mice. It is noted that the median intraperitoneal LD₅₀ dose of Dysport^{®} in mice is not necessarily the same as the median intraperitoneal LD₅₀ dose of another botulinum neurotoxin-containing product, such as Botox^{®}, as different pharmaceutical preparations are produced differently. For example, units of Botox^{®}, as described in the art, are not the same as the units of the present invention, which are units of Dysport^{®}.

Dysport^{®} is an injectable form of botulinum toxin type A (BoNT-A), which is isolated and purified from Clostridium bacteria producing BoNT-A. It is supplied as a lyophilized powder. Dysport^{®} has approved therapeutic indications in the United States for the treatment of adults with Cervical Dystonia (CD), the treatment of Upper Limb Spasticity (ULS) in adult patients, and the treatment of lower limb spasticity in children to improve tone and spasticity. The medicine was first registered in the United Kingdom in 1990 for other uses and is licensed in more than 80 countries in eight different indications, with over 1,300 peer-reviewed publications.

In use, botulinum neurotoxin may also be administered bilaterally. The term "bilateral," and its variants, when used herein in reference to administration, refers to the administration of botulinum neurotoxin to both legs of the subject.

Thus, in use, botulinum neurotoxin may be administered to a subject bilaterally in an amount of at least about 5 units per kilogram of the total body weight of the subject per leg. In use, botulinum neurotoxin may be administered to a subject bilaterally in an amount of at least about 10 units per kilogram of the total body weight of the subject per leg. The amount of botulinum neurotoxin administered to a subject bilaterally may be less than about 15 units per kilogram of the total body weight of the subject per leg (e.g. less than about 12 units per kilogram of the total body weight of the subject per leg).

In use, botulinum neurotoxin may be administered bilaterally to each leg of the subject in an amount of 10 to 15 units, 11 to 14 units, or 12 to 13 units per kilogram of the total body weight of the subject per leg.

In use, the total amount of botulinum neurotoxin administered to the subject per treatment session may be 1000 units or less, 900 units or less, 800 units or less, 700 units or less, 600 units or less, or 500 units or less.

In some uses, subsequent treatment with botulinum neurotoxin following a previous such treatment may not take place within 12 weeks of the previous treatment. For example, the subsequent treatment may take place 16 to 22 weeks after the previous treatment.

In use, botulinum neurotoxin may be administered by any means, for example parenterally, intra-muscularly, sub-cutaneously, intradermally, or transdermally. In use, administration may be by injection to a muscle.

In use, administration may, for example, be to the muscles of the gastrocnemius-soleus complex. For example, in use, botulinum neurotoxin may be administered to a gastrocnemius of the subject. In use, botulinum neurotoxin may be administered to an individual gastrocnemius in an amount of 6 to 9 units or 7 to 8 units per kilogram of the total body weight of the subject. In use, each gastrocnemius of the subject may be administered with such an amount. In use, botulinum neurotoxin may also or alternatively be administered to a soleus of the subject. In use, botulinum neurotoxin may be administered to an individual soleus in an amount of 4 to 6 units or about 5 units per kilogram of the total body weight of the subject. In use, each soleus of the subject may be administered with such an amount.

In use, administration may also take place at more than one site in any single muscle. In certain uses, no more than 0.5 mL, 0.25 mL, or 0.1 mL of Dysport^{®} may be administered at any single site.

The amount of botulinum neurotoxin to be administered in use may, for example be determined based in part on factors such as: the weight of the subject, whether administration is to be unilateral or bilateral, whether administration will be to the gastrocnemius and/or to the soleus, the severity of the subject's spasticity, what muscles are affected by the spasticity, the presence of local muscle weakness, and/or the subject's history with treatment with botulinum neurotoxin.

For example, in use, when administration is unilateral, a total dosage of 10 to 15 units/kg of the total body weight of the subject may be administered. As such, 100 to 150 units may be administered to a patient having a total body weight of 10 kg, 200 to 300 units may be administered to a patient having a total body weight of 20 kg, 300 to 450 units may be administered to a patient having a total body weight of 30 kg, 400 to 600 units may be administered to a patient having a total body weight of 40 kg, 500 to 750 units may be administered to a patient having a total body weight of 50 kg, 600 to 900 units may be administered to a patient having a total body weight of 60 kg, and 700 to 1,050 units may be administered to a patient having a total body weight of 70 kg.

In use, when administration is bilateral, for example, a total dosage of 20 to 30 units/kg of the total body weight of the subject may be administered. As such, 200 to 300 units may be administered to a patient having a total body weight of 10 kg, 400 to 600 units may be administered to a patient having a total body weight of 20 kg, 600 to 900 units may be administered to a patient having a total body weight of 30 kg, 800 to 1,200 units may be administered to a patient having a total body weight of 40 kg, 1,000 to 1,500 units may be administered to a patient having a total body weight of 50 kg, 1,200 to 1,800 units may be administered to a patient having a total body weight of 60 kg, and 1,400 to 2,100 units may be administered to a patient having a total body weight of 70 kg.

In use, when a gastrocnemius of the subject is affected by spasticity, administration of botulinum neurotoxin may, for example, be to the gastrocnemius.

In use, wherein administration is unilateral, administration to the soleus may, for example, be 6 to 9 unitslkg of the total body weight of the subject. For example, 60 to 90 units may be administered to a patient having a total body weight of 10 kg, 120 to 180 units may be administered to a patient having a total body weight of 20 kg, 180 to 270 units may be administered to a patient having a total body weight of 30 kg, 240 to 360 units may be administered to a patient having a total body weight of 40 kg, 300 to 450 units may be administered to a patient having a total body weight of 50 kg, 360 to 540 units may be administered to a patient having a total body weight of 60 kg, and 420 to 630 units may be administered to a patient having a total body weight of 70 kg. In use, wherein administration is bilateral, the above amounts may be individually administered to the gastrocnemius of each leg in the above amounts.

In use, when a soleus of the subject is affected by spasticity, administration of botulinum neurotoxin may, for example, be to the soleus. In use, wherein administration is unilateral, administration to the soleus may, for example, be 4 to 6 units/kg of the total body weight of the subject. For example, 40 to 60 units may be administered to a patient having a total body weight of 10 kg, 80 to 120 units may be administered to a patient having a total body weight of 20 kg, 120 to 180 units may be administered to a patient having a total body weight of 30 kg, 160 to 240 units may be administered to a patient having a total body weight of 40 kg, 200 to 300 units may be administered to a patient having a total body weight of 50 kg, 240 to 360 units may be administered to a patient having a total body weight of 60 kg, and 280 to 420 units may be administered to a patient having a total body weight of 70 kg. In use, wherein administration is bilateral, the above amounts may be individually administered to the soleus of each leg in the above amounts.

The subject may be a subject having spasticity in association with equinus, cerebral palsy, spinal cord injury, multiple sclerosis, stroke, brain trauma, head trauma or combinations thereof. In one embodiment a subject has equinus. Preferably a subject has spasticity and equinus. Equinus may be dynamic equinus (e.g. dynamic equinus foot deformity).

The level of a patient's spasticity may, for example, be measured using the Modified Ashworth Scale (MAS) which measures the level of increase in resistance during passive soft-tissue stretching. A MAS score of 0 indicates no increase in muscle tone. A score of 1 indicates a slight increase in muscle tone, manifested by a catch and release or by minimal resistance at the end of the range of motion when the affected part(s) is moved in flexion or extension. A score of 1+ indicates a slight increase in muscle tone, manifested by a catch, followed by minimal resistance throughout the remainder (less than half) of the range of movement. A score of 2 indicates a more marked increase in muscle tone through most of the range of motion, but the affected part(s) easily moved. A score of 3 indicates considerable increase in muscle tone with passive movement being difficult. A score of 4 indicates that the affected part is rigid in flexion or extension.

In use, the amount of units administered to the subject may be directly proportional to the level of the subject's spasticity.

For example, in use, when a subject is suffering from severe spasticity in his/her gastrocnemius, as indicated by a MAS score of 3 or above, administration may, for example, be 7.5 to 9 unitslkg of the total body weight of the subject per leg (*i*.*e*. 7.5 to 9 unitslkg for unilateral administration and 15 to 18 units/kg for bilateral administration). In use, administration may be 8 to 9 unitslkg per leg, 8.5 to 9 unitslkg per leg, or 8.75 to 9 unitslkg per leg.

For example, in use, when a subject is suffering from severe spasticity in his/her soleus, as indicated by a MAS score of 3 or above, administration may, for example, be 5 to 6 units/kg of the total body weight of the subject per leg (*i*.*e*. 5 to 6 units/kg for unilateral administration and 10 to 12 unitslkg for bilateral administration). In use, administration may be 5.5 to 6 unitslkg per leg or 5.75 to 6 units/kg per leg.

By contrast, in use, when a subject is suffering from less severe or minor spasticity in his/her gastrocnemius, administration may, for example, be 6 to 7.5 units/kg of the total body weight of the subject per leg (*i*.*e*. 6 to 7.5 units/kg for unilateral administration and 12 to 15 unitslkg for bilateral administration). In use, administration may be 6 to 7 units/kg per leg, 6 to 6.5 unitslkg per leg, or 6 to 6.25 unitslkg per leg.

In use, when a subject is suffering from less severe or minor spasticity in his/her soleus, administration may, for example, be 4 to 5 units/kg of the total body weight of the subject per leg (*i.e*. 4 to 5 unitslkg for unilateral administration and 8 to 10 unitslkg for bilateral administration). In use, administration may be 4 to 4.5 units/kg per leg, or 4 to 4.25 units/kg per leg.

In addition, in use, a lower dose may be administered if the subject has exhibited adverse effects following previous treatment with botulinum neurotoxin or exhibits local muscle weakness in the muscle to which the botulinum neurotoxin is to be administered. Examples of adverse effects include respiratory tract infection, nasopharyngitis, influenza, pharyngitis, cough, pyrexia, bronchitis, rhinitis, varicella, ear infection, gastroenteritis, vomiting, nausea, oropharyngeal pain, pain in extremities, muscular weakness, convulsion, and epilepsy. An example of such a lower dose is 10 to 12.5 units/kg of the total body weight of the subject per leg (*i.e*. 10 to 12.5 unitslkg for unilateral administration and 20 to 25 units/kg for bilateral administration). Other examples include 10 to 12, 10 to 11.5, 10 to 11, and 10 to 10.5 unitslkg of the total body weight of the subject per leg. For administration to the gastrocnemius, the dosage may, for example, be 6 to 7.5 units/kg, 6 to 7 units/kg, or 6 to 6.5 unitslkg per leg. For administration to the soleus, the dosage may, for example, be 4 to 5 units/kg or 4 to 4.5 unitslkg per leg.

Alternatively, in use, a higher dose may be administered if the subject has not exhibited a desired response to previous treatment with botulinum neurotoxin. A subject's response may, for example, be measured using the Physician's Global Assessment (PGA) of response to treatment. The PGA measures response on a scale from -4 to +4, with -4 indicating that response is markedly worse, to 0, which indicates no change, to +4 indicating that response has markedly improved. For example, a desired response may be a PGA score following treatment of 0.25 or above, 0.5 or above, 1 or above, 2 or above, 3 or above, or 4.

An example of such a higher dose is 12.5 to 15 units/kg of the total body weight of the subject per leg (*i*.*e*. 12.5 to 15 unitslkg for unilateral administration and 25 to 30 units/kg for bilateral administration). Other examples include 13 to 15, 13.5 to 15, 14 to 15, and 14.5 to 15 unitslkg of the total body weight of the subject per leg. For administration to the gastrocnemius, the dosage in use may, for example, be 7.5 to 9 units/kg per leg. For administration to the soleus, the dosage in use may, for example, be 5 to 6 units/kglleg.

In use, in the event of local muscle weakness in a particular muscle, the administration may be to a different muscle. For example, if the subject is suffering from local muscle weakness in a gastrocnemius, administration may be to the subject's other gastrocnemius, or to a soleus, or to some other appropriate muscle. Similarly, if the subject is suffering from local muscle weakness in a soleus, administration may be to the subject's other soleus, or to a gastrocnemius, or to some other appropriate muscle.

In use, whether administration is to be unilateral or bilateral depends on various factors, including the muscles affected by the patient's spasticity, the severity of the subject's spasticity, the presence of local muscle weakness, and/or the subject's history with treatment with botulinum neurotoxin. For example, administration may be unilateral if only one leg is affected by spasticity and bilateral if both legs are affected. Also, a subject may have spasticity in only one leg but the spasticity may be severe enough that bilateral administration may be appropriate. By contrast, a subject may have spasticity in both legs but local muscle weakness in one leg may mean that unilateral administration to only the other leg is appropriate. Also, unilateral rather than bilateral administration may be appropriate in instances where the subject has had adverse effects resulting from previous treatment with botulinum neurotoxin and bilateral rather than unilateral administration may be appropriate in instances where the subject has not exhibited a desired response following previous unilateral and/or lower dose treatment with botulinum neurotoxin.

In use, the botulinum neurotoxin may be administered as part of a method of treating a subject for lower limb spasticity comprising: (A) obtaining the weight of the subject; (B) choosing unilateral or bilateral administration; (C) based on the weight of the patient and whether administration is to be unilateral or bilateral, determining the amount of botulinum neurotoxin to administer to the subject; and (D) administering botulinum neurotoxin in such an amount to the subject. In determining the appropriate amount of botulinum neurotoxin, the method may also involve considering whether administration will be to the gastrocnemius and/or to the soleus, the amount of the subject's spasticity, what muscles are affected by the spasticity, the presence of local muscle weakness, the subject's history with treatment with botulinum neurotoxin, the subject's age, the subject's height, the subject's body surface area, and/or the subject's sex. In determining an appropriate dosage amount, adjustments may be made as described above in view of the aforementioned factors.

The invention relates to a tool for use in determining a dosage amount of a botulinum neurotoxin to be administered to
a subject, the tool comprising:
   a first member having thereon a first row of numbers indicative of the weight of the subject and a second row of numbers indicative of a dosage amount of the botulinum neurotoxin to be administered to the subject, wherein the numbers in the first row indicating a specific weight are in latitudinal or radial alignment with corresponding numbers in the second row indicating the specific dosage amount to be administered to a subject having that specific weight, wherein the dosage amount corresponds to 5 to 15 units per kilogram of the total body weight of the subject per leg; and
   a second member;
wherein the first and second members are in a relationship with one another such that one member may be moved by a user to provide an indication as to the dosage amount of the botulinum neurotoxin to be administered to a subject having a particular weight; and
   wherein the first member further has thereon a row of numbers indicative of the dosage amount of the botulinum neurotoxin to be administered to a particular muscle of the subject and the numbers in the row which indicate the specific dosage amount to be administered to the muscle of a subject having a specific weight are in longitudinal or radial alignment with corresponding numbers in the first row indicating that specific weight, wherein the dosage amount corresponds to 6 to 9 units per kilogram of the total body weight of the subject to be administered to an individual gastrocnemius muscle of the subject or wherein the dosage amount corresponds to 4 to 6 units per kilogram of the total body weight of the subject to be administered to an individual soleus muscle of the subject.

In some embodiments, both first (1) and second (4) members are planar surfaces and are in longitudinal sliding arrangement with each other. The numbers in the first row (2) are in latitudinal alignment with corresponding numbers in the second row (3). The second member overlays the first and contains an opening (5) that allows for the rows on the first member to be viewed. In certain embodiments, the second member contains two or more openings (6) with each opening allowing for one row on the first member to be viewed. One member may be slid in relation to the other with the numbers viewable through the opening or openings indicating a specific weight and the dosage amount to be administered to a subject having that specific weight. In certain embodiments, the first planar surface folds around and encloses the second planar surface.

In other embodiments, the first member (1) is a planar surface and the second member (4) is a cursor in a longitudinal sliding arrangement with respect to the first member. The numbers in the first row (2) are in latitudinal alignment with corresponding numbers in the second row (3). The cursor may be slid along the first member such that it indicates a specific weight and the dosage amount to be administered to a subject having that specific weight. The indication may be by way of a line (7) on the cursor which overlays a specific weight and the dosage amount to be administered to a subject having that specific weight. In such an embodiment, the cursor may, for example, be transparent or translucent to allow for the user to see the numbers underneath the line. Alternatively, the cursor may contain an opening (5) or openings (6) which allow for the numbers on the first member to be viewable by the user.

In yet other embodiments, the first member (1) is a circular planar surface and the second member (4) is a dial that pivots around the center point of the first member. The numbers in the first row (2) are in radial alignment with corresponding numbers in the second row (3). The cursor may be pivoted such that it indicates a specific weight and the dosage amount to be administered to a subject having that specific weight. The indication may be by way of a line (7) on the cursor which overlays a specific weight and the dosage amount to be administered to a subject having that specific weight. In such an embodiment, the cursor may, for example, be transparent or translucent to allow for the user to see the numbers underneath the line. Alternatively, the cursor may contain an opening (5) or openings (6) which allow for the numbers on the first member to be viewable by the user.

In an embodiment, one row of numbers indicates the dosage amount of the active agent to be administered to the gastrocnemius of the leg (9) and another row indicates the dosage amount of the active agent to be administered to the soleus of the leg (10).

In embodiments of the tool that contain an opening (5) or two or more openings (6) in the second member, the opening may contain a window made of transparent or translucent material.

In embodiments wherein the second member contains two or more openings (6), each opening may correspond to a specific row on the first member. For example, one opening may allow for the numbers of the first row (2) to be viewed, another opening may allow for the numbers of the second row (3) to be viewed, and third and fourth openings may allow for the numbers of the additional rows (8) to be viewed.

In embodiments wherein the second member (4) contains an opening (5) or openings (6) through which the numbers of the rows on the first member (1) may be viewed or wherein the second member is transparent or translucent, the second member may contain a first marking (11) aligned with the row of numbers which indicate the weight of the subject (2), a second marking (12) aligned with the row of numbers which indicate the dosage amount of the active agent to be administered to the subject (3), and third (13) and fourth markings (14) aligned respectively with the row indicating the dosage amount of the active agent to be administered to the gastrocnemius of the subject's leg (9) and the row indicating the dosage amount of the active agent to be administered to the soleus of the subject's leg (10). The markings may identify each row respectively, for example as: "Total Weight of the Subject"; "Amount of Agent Per Leg to be Administered to Subject"; "Amount of Agent to be Administered Per Leg to the Gastrocnemius of the Subject"; and "amount of Agent to be Administered Per Leg to the Soleus of the Subject." In embodiments wherein the second member is transparent or translucent, the markings may be on a label (15) to ensure better visibility.

In use, a botulinum neurotoxin may be part of a composition comprising an effective amount of botulinum neurotoxin. The composition may comprise a diluent, for example a sodium chloride solution. The solution may, for example, be a 0.9% sodium chloride solution.

The composition may comprise 1 to 1,000 units, 1 to 750 units, 1 to 500 units, 1 to 400 units, 1 to 300 units, or 1 to 200 units of botulinum neurotoxin.

The composition may comprise botulinum neurotoxin in the amount of 500 units and 1 to 5 mL of sodium chloride solution, for example 1, 2, 2.5, and 5 mL of the solution. The composition may comprise 500 units and 2.5 mL of solution.

The composition may comprise botulinum neurotoxin in the amount of 300 units and 1 to 3 mL of sodium chloride solution, for example 1, 1.5, 2.5, and 3 mL of the solution.

The composition may be formed by reconstituting lyophilized botulinum neurotoxin in a solution, for example the aforementioned sodium chloride solution. Following reconstitution, the composition may be stored under refrigeration at 2 to 8 °C and protected from light.

In use, the amount of the composition administered may be the amount determined to be appropriate as described above. For example, for a composition comprising 500 units diluted in 2.5 mL of solution, each 0.1 mL of the composition will contain 20 units. As such, the amount of the composition to be administered to a subject in a unilateral treatment may be 0.05 to 0.075 mL (containing 10 to 15 units) per kg of the total body weight of the subject. Thus, for a subject weighing 50 kg, 2.5 to 3.75 mL of the composition may be administered. The amount of the composition to be administered to a subject in a bilateral treatment may be 0.1 to 0.15 mL (containing 20 to 30 units) per kg of the total body weight of the subject. For a subject weighting 50 kg, 5 to 7.5 mL of the composition may be administered.

The composition may further comprise human serum albumin. For example, 1 to 100 mg/ml, 1 to 50 mg/ml, 1 to 25 mg/ml, or 25 mg/ml.

The composition may further comprise an excipient. The excipient may, for example, be a filler, a binder, a disintegrant, an anti-adherent, a solvent, a buffering agent, a preservative, or a humectant. Examples of fillers include cellulose, lactose, sucrose, glucose, mannose, and sorbitol. The composition may comprise, for example, 10 mg/ml lactose. Examples of binders include gelatin, cellulose, polyvinyl pyrrolidone, starch, and sucrose. Examples of disintegrants include polyvinyl pyrrolidone and carboxymethyl cellulose. Examples of preservatives include parabens. Examples of solvents include water, oils, glycerol, propylene glycol, and ethanol. Examples of buffering agents include phosphates, carbonates, citrates, and lactates. Examples of humectants include glycerol, ethylene glycol, and polyethylene glycol (PEG).

### SEQUENCE LISTING

SEQ ID NO: 1 (BoNT/A - UniProt P10845)
SEQ ID NO: 2 (BoNTIB - UniProt P10844) 
MPVTINNFNYNDPIDNNNIIMMEPPFARGTGRYYKAFKITDRIWIIPERYTFGYKPEDFN
KSSGIFNRDVCEYYDPDYLNTNDKKNIFLQTMIKLFNRIKSKPLGEKLLEMIINGIPYLG
DRRVPLEEFNTNIASVTVNKLISNPGEVERKKGIFANLIIFGPGPVLNENETIDIGIQNH
FASREGFGGIMQMKFCPEYVSVFNNVQENKGASIFNRRGYFSDPALILMHELIHVLHGLY
GIKVDDLPIVPNEKKFFMQSTDAIQAEELYTFGGQDPSIITPSTDKSIYDKVLQNFRGIV
DRLNKVLVCISDPNININIYKNKFKDKYKFVEDSEGKYSIDVESFDKLYKSLMFGFTETN
IAENYKIKTRASYFSDSLPPVKIKNLLDNEIYTIEEGFNISDKDMEKEYRGQNKAINKQA
YEEISKEHLAVYKIQMCKSVKAPGICIDVDNEDLFFIADKNSFSDDLSKNERIEYNTQSN
YIENDFPINELILDTDLISKIELPSENTESLTDFNVDVPVYEKQPAIKKIFTDENTIFQY
LYSQTFPLDIRDISLTSSFDDALLFSNKVYSFFSMDYIKTANKVVEAGLFAGWVKQIVND
 
FVIEANKSNTMDKIADISLIVPYIGLALNVGNETAKGNFENAFEIAGASILLEFIPELLI
PVVGAFLLESYIDNKNKIIKTIDNALTKRNEKWSDMYGLIVAQWLSTVNTQFYTIKEGMY
KALNYQAQALEEIIKYRYNIYSEKEKSNINIDFNDINSKLNEGINQAIDNINNFINGCSV
SYLMKKMIPLAVEKLLDFDNTLKKNLLNYIDENKLYLIGSAEYEKSKVNKYLKTIMPFDL
SIYTNDTILIEMFNKYNSEILNNIILNLRYKDNNLIDLSGYGAKVEVYDGVELNDKNQFK
LTSSANSKIRVTQNQNIIFNSVFLDFSVSFWIRIPKYKNDGIQNYIHNEYTIINCMKNNS
GWKISIRGNRIIWTLIDINGKTKSVFFEYNIREDISEYINRWFFVTITNNLNNAKIYING
KLESNTDIKDIREVIANGEIIFKLDGDIDRTQFIWMKYFSIFNTELSQSNIEERYKIQSY
SEYLKDFWGNPLMYNKEYYMFNAGNKNSYIKLKKDSPVGEILTRSKYNQNSKYINYRDLY
IGEKFIIRRKSNSQSINDDIVRKEDYIYLDFFNLNQEWRVYTYKYFKKEEEKLFLAPISD
SDEFYNTIQIKEYDEQPTYSCQLLFKKDEESTDEIGLIGIHRFYESGIVFEEYKDYFCIS
KWYLKEVKRKPYNLKLGCNWQFIPKDEGWTE
SEQ ID NO: 3 (BoNT/C - UniProt P18640) 
MPITINNFNYSDPVDNKNILYLDTHLNTLANEPEKAFRITGNIWVIPDRFSRNSNPNLNK
PPRVTSPKSGYYDPNYLSTDSDKDPFLKEIIKLFKRINSREIGEELIYRLSTDIPFPGNN
NTPINTFDFDVDFNSVDVKTRQGNNWVKTGSINPSVIITGPRENIIDPETSTFKLTNNTF
AAQEGFGALSIISISPRFMLTYSNATNDVGEGRFSKSEFCMDPILILMHELNHAMHNLYG
IAIPNDQTISSVTSNIFYSQYNVKLEYAEIYAFGGPTIDLIPKSARKYFEEKALDYYRSI
AKRLNSITTANPSSFNKYIGEYKQKLIRKYRFVVESSGEVTVNRNKFVELYNELTQIFTE
FNYAKIYNVQNRKIYLSNVYTPVTANILDDNVYDIQNGFNIPKSNLNVLFMGQNLSRNPA
LRKVNPENMLYLFTKFCHKAIDGRSLYNKTLDCRELLVKNTDLPFIGDISDVKTDIFLRK
DINEETEVIYYPDNVSVDQVILSKNTSEHGQLDLLYPSIDSESEILPGENQVFYDNRTQN
VDYLNSYYYLESQKLSDNVEDFTFTRSIEEALDNSAKVYTYFPTLANKVNAGVQGGLFLM
WANDVVEDFTTNILRKDTLDKISDVSAIIPYIGPALNISNSVRRGNFTEAFAVTGVTILL
EAFPEFTIPALGAFVIYSKVQERNEIIKTIDNCLEQRIKRWKDSYEWMMGTWLSRIITQF
NNISYQMYDSLNYQAGAIKAKIDLEYKKYSGSDKENIKSQVENLKNSLDVKISEAMNNIN
KFIRECSVTYLFKNMLPKVIDELNEFDRNTKAKLINLIDSHNIILVGEVDKLKAKVNNSF
QNTIPFNIFSYTNNSLLKDIINEYFNNINDSKILSLQNRKNTLVDTSGYNAEVSEEGDVQ
LNPIFPFDFKLGSSGEDRGKVIVTQNENIVYNSMYESFSISFWIRINKWVSNLPGYTIID
SVKNNSGWSIGIISNFLVFTLKQNEDSEQSINFSYDISNNAPGYNKWFFVTVTNNMMGNM
KIYINGKLIDTIKVKELTGINFSKTITFEINKIPDTGLITSDSDNINMWIRDFYIFAKEL
DGKDINILFNSLQYTNVVKDYWGNDLRYNKEYYMVNIDYLNRYMYANSRQIVFNTRRNNN
DFNEGYKIIIKRIRGNTNDTRVRGGDILYFDMTINNKAYNLFMKNETMYADNHSTEDIYA
IGLREQTKDINDNIIFQIQPMNNTYYYASQIFKSNFNGENISGICSIGTYRFRLGGDWYR
HNYLVPTVKQGNYASLLESTSTHWGFVPVSE
SEQ ID NO: 4 (BoNT/D - UniProt P19321) 
MTWPVKDFNYSDPVNDNDILYLRIPQNKLITTPVKAFMITQNIWVIPERFSSDTNPSLSK
PPRPTSKYQSYYDPSYLSTDEQKDTFLKGIIKLFKRINERDIGKKLINYLVVGSPFMGDS
STPEDTFDFTRHTTNIAVEKFENGSWKVTNIITPSVLIFGPLPNILDYTASLTLQGQQSN
PSFEGFGTLSILKVAPEFLLTFSDVTSNQSSAVLGKSIFCMDPVIALMHELTHSLHQLYG
INIPSDKRIRPQVSEGFFSQDGPNVQFEELYTFGGLDVEIIPQIERSQLREKALGHYKDI
AKRLNNINKTIPSSWISNIDKYKKIFSEKYNFDKDNTGNFVVNIDKFNSLYSDLTNVMSE
VVYSSQYNVKNRTHYFSRHYLPVFANILDDNIYTIRDGFNLTNKGFNIENSGQNIERNPA
LQKLSSESVVDLFTKVCLRLTKNSRDDSTCIKVKNNRLPYVADKDSISQEIFENKIITDE
TNVQNYSDKFSLDESILDGQVPINPEIVDPLLPNVNMEPLNLPGEEIVFYDDITKYVDYL
NSYYYLESQKLSNNVENITLTTSVEEALGYSNKIYTFLPSLAEKVNKGVQAGLFLNWANE
VVEDFTTNIMKKDTLDKISDVSVIIPYIGPALNIGNSALRGNFNQAFATAGVAFLLEGFP
 
EFTIPALGVFTFYSSIQEREKIIKTIENCLEQRVKRWKDSYQWMVSNWLSRITTQFNHIN
YQMYDSLSYQADAIKAKIDLEYKKYSGSDKENIKSQVENLKNSLDVKISEAMNNINKFIR
ECSVTYLFKNMLPKVIDELNKFDLRTKTELINLIDSHNIILVGEVDRLKAKVNESFENTM
PFNIFSYTNNSLLKDIINEYFNSINDSKILSLQNKKNALVDTSGYNAEVRVGDNVQLNTI
YTNDFKLSSSGDKIIVNLNNNILYSAIYENSSVSFWIKISKDLTNSHNEYTIINSIEQNS
GWKLCIRNGNIEWILQDVNRKYKSLIFDYSESLSHTGYTNKWFFVTITNNIMGYMKLYIN
GELKQSQKIEDLDEVKLDKTIVFGIDENIDENQMLWIRDFNIFSKELSNEDINIVYEGQI
LRNVIKDYWGNPLKFDTEYYIINDNYIDRYIAPESNVLVLVQYPDRSKLYTGNPITIKSV
SDKNPYSRILNGDNIILHMLYNSRKYMIIRDTDTIYATQGGECSQNCVYALKLQSNLGNY
GIGIFSIKNIVSKNKYCSQIFSSFRENTMLLADIYKPWRFSFKNAYTPVAVTNYETKLLS
TSSFWKFISRDPGWVE
SEQ ID NO: 5 (BoNT/E - UniProt Q00496) 
MPKINSFNYNDPVNDRTILYIKPGGCQEFYKSFNIMKNIWIIPERNVIGTTPQDFHPPTS
LKNGDSSYYDPNYLQSDEEKDRFLKIVTKIFNRINNNLSGGILLEELSKANPYLGNDNTP
DNQFHIGDASAVEIKFSNGSQDILLPNVIIMGAEPDLFETNSSNISLRNNYMPSNHRFGS
IAIVTFSPEYSFRFNDNCMNEFIQDPALTLMHELIHSLHGLYGAKGITTKYTITQKQNPL
ITNIRGTNIEEFLTFGGTDLNIITSAQSNDIYTNLLADYKKIASKLSKVQVSNPLLNPYK
DVFEAKYGLDKDASGIYSVNINKFNDIFKKLYSFTEFDLRTKFQVKCRQTYIGQYKYFKL
SNLLNDSIYNISEGYNINNLKVNFRGQNANLNPRIITPITGRGLVKKIIRFCKNIVSVKG
IRKSICIEINNGELFFVASENSYNDDNINTPKEIDDTVTSNNNYENDLDQVILNFNSESA
PGLSDEKLNLTIQNDAYIPKYDSNGTSDIEQHDVNELNVFFYLDAQKVPEGENNVNLTSS
IDTALLEQPKIYTFFSSEFINNVNKPVQAALFVSWIQQVLVDFTTEANQKSTVDKIADIS
IVVPYIGLALNIGNEAQKGNFKDALELLGAGILLEFEPELLIPTILVFTIKSFLGSSDNK
NKVIKAINNALKERDEKWKEVYSFIVSNWMTKINTQFNKRKEQMYQALQNQVNAIKTIIE
SKYNSYTLEEKNELTNKYDIKQIENELNQKVSIAMNNIDRFLTESSISYLMKIINEVKIN
KLREYDENVKTYLLNYIIQHGSILGESQQELNSMVTDTLNNSIPFKLSSYTDDKILISYF
NKFFKRIKSSSVLNMRYKNDKYVDTSGYDSNININGDVYKYPTNKNQFGIYNDKLSEVNI
SQNDYIIYDNKYKNFSISFWVRIPNYDNKIVNVNNEYTIINCMRDNNSGWKVSLNHNEII
WTFEDNRGINQKLAFNYGNANGISDYINKWIFVTITNDRLGDSKLYINGNLIDQKSILNL
GNIHVSDNILFKIVNCSYTRYIGIRYFNIFDKELDETEIQTLYSNEPNTNILKDFWGNYL
LYDKEYYLLNVLKPNNFIDRRKDSTLSINNIRSTILLANRLYSGIKVKIQRVNNSSTNDN
LVRKNDQVYINFVASKTHLFPLYADTATTNKEKTIKISSSGNRFNQVVVMNSVGNCTMNF
KNNNGNNIGLLGFKADTVVASTWYYTHMRDHTNSNGCFWNFISEEHGWQEK
SEQ ID NO: 6 (BoNT/F - UniProt P30996) 
MPVAINSFNYNDPVNDDTILYMQIPYEEKSKKYYKAFEIMRNVWIIPERNTIGTNPSDFD
PPASLKNGSSAYYDPNYLTTDAEKDRYLKTTIKLFKRINSNPAGKVLLQEISYAKPYLGN
DHTPIDEFSPVTRTTSVNIKLSTNVESSMLLNLLVLGAGPDIFESCCYPVRKLIDPDVVY
DPSNYGFGSINIVTFSPEYEYTFNDISGGHNSSTESFIADPAISLAHELIHALHGLYGAR
GVTYEETIEVKQAPLMIAEKPIRLEEFLTFGGQDLNIITSAMKEKIYNNLLANYEKIATR
LSEVNSAPPEYDINEYKDYFQWKYGLDKNADGSYTVNENKFNEIYKKLYSFTESDLANKF
KVKCRNTYFIKYEFLKVPNLLDDDIYTVSEGFNIGNLAVNNRGQSIKLNPKIIDSIPDKG
LVEKIVKFCKSVIPRKGTKAPPRLCIRVNNSELFFVASESSYNENDINTPKEIDDTTNLN
NNYRNNLDEVILDYNSQTIPQISNRTLNTLVQDNSYVPRYDSNGTSEIEEYDVVDFNVFF
YLHAQKVPEGETNISLTSSIDTALLEESKDIFFSSEFIDTINKPVNAALFIDWISKVIRD
FTTEATQKSTVDKIADISLIVPYVGLALNIIIEAEKGNFEEAFELLGVGILLEFVPELTI
PVILVFTIKSYIDSYENKNKAIKAINNSLIEREAKWKEIYSWIVSNWLTRINTQFNKRKE
QMYQALQNQVDAIKTAIEYKYNNYTSDEKNRLESEYNINNIEEELNKKVSLAMKNIERFM
 
TESSISYLMKLINEAKVGKLKKYDNHVKSDLLNYILDHRSILGEQTNELSDLVTSTLNSS
IPFELSSYTNDKILIIYFNRLYKKIKDSSILDMRYENNKFIDISGYGSNISINGNVYIYS
TNRNQFGIYNSRLSEVNIAQNNDIIYNSRYQNFSISFWVRIPKHYKPMNHNREYTIINCM
GNNNSGWKISLRTVRDCEIIWTLQDTSGNKENLIFRYEELNRISNYINKWIFVTITNNRL
GNSRIYINGNLIVEKSISNLGDIHVSDNILFKIVGCDDETYVGIRYFKVFNTELDKTEIE
TLYSNEPDPSILKNYWGNYLLYNKKYYLFNLLRKDKYITLNSGILNINQQRGVTEGSVFL
NYKLYEGVEVIIRKNGPIDISNTDNFVRKNDLAYINVVDRGVEYRLYADTKSEKEKIIRT
SNLNDSLGQIIVMDSIGNNCTMNFQNNNGSNIGLLGFHSNNLVASSWYYNNIRRNTSSNG
CFWSSISKENGWKE
SEQ ID NO: 7 (BoNT/G - UniProt Q60393)

MPVNIKXFNYNDPINNDDIIMMEPFNDPGPGTYYKAFRIIDRIWIVPERFTYGFQPDQFN
ASTGVFSKDVYEYYDPTYLKTDAEKDKFLKTMIKLFNRINSKPSGQRLLDMIVDAIPYLG
NASTPPDKFAANVANVSINKKIIQPGAEDQIKGLMTNLIIFGPGPVLSDNFTDSMIMNGH
SPISEGFGARMMIRFCPSCLNVFNNVQENKDTSIFSRRAYFADPALTLMHELIHVLHGLY
GIKISNLPITPNTKEFFMQHSDPVQAEELYTFGGHDPSVISPSTDMNIYNKALQNFQDIA
NRLNIVSSAQGSGIDISLYKQIYKNKYDFVEDPNGKYSVDKDKFDKLYKALMFGFTETNL
AGEYGIKTRYSYFSEYLPPIKTEKLLDNTIYTQNEGFNIASKNLKTEFNGQNKAVNKEAY
EEISLEHLVIYRIAMCKPVMYKNTGKSEQCIIVNNEDLFFIANKDSFSKDLAKAETIAYN
TQNNTIENNFSIDQLILDNDLSSGIDLPNENTEPFTNFDDIDIPVYIKQSALKKIFVDGD
SLFEYLHAQTFPSNIENLQLTNSLNDALRNNNKVYTFFSTNLVEKANTVVGASLFVNWVK
GVIDDFTSESTQKSTIDKVSDVSIIIPYIGPALNVGNETAKENFKNAFEIGGAAILMEFI
PELIVPIVGFFTLESYVGNKGHIIMTISNALKKRDQKWTDMYGLIVSQWLSTVNTQFYTI
KERMYNALNNQSQAIEKIIEDQYNRYSEEDKMNINIDFNDIDFKLNQSINLAINNIDDFI
NQCSISYLMNRMIPLAVKKLKDFDDNLKRDLLEYIDTNELYLLDEVNILKSKVNRHLKDS
IPFDLSLYTKDTILIQVFNNYISNISSNAILSLSYRGGRLIDSSGYGATMNVGSDVIFND
IGNGQFKLNNSENSNITAHQSKFVVYDSMFDNFSINFWVRTPKYNNNDIQTYLQNEYTII
SCIKNDSGWKVSIKGNRIIWTLIDVNAKSKSIFFEYSIKDNISDYINKWFSITITNDRLG
NANIYINGSLKKSEKILNLDRINSSNDIDFKLINCTDTTKFVWIKDFNIFGRELNATEVS
SLYWIQSSTNTLKDFWGNPLRYDTQYYLFNQGMQNIYIKYFSKASMGETAPRTNFNNAAI
NYQNLYLGLRFIIKKASNSRNINNDNIVREGDYIYLNIDNISDESYRVYVLVNSKEIQTQ
LFLAPINDDPTFYDVLQIKKYYEKTTYNCQILCEKDTKTFGLFGIGKFVKDYGYVWDTYD
NYFCISQWYLRRISENINKLRLGCNWQFIPVDEGWTE

### EXAMPLES

### Example 1

The efficacy of botulinum neurotoxin in treating lower limb spasticity was evaluated in a double-blind, placebo-controlled multicenter study in patients 2 to 17 years of age suffering from spasticity because of cerebral palsy causing dynamic equinus foot deformity.

A total of 235 toxin naive or non-naive patients with a Modified Ashworth Score of grade 2 or greater at the ankle plantar flexor were enrolled to receive Dysport^{®} or placebo.

Seventy-nine patients received 10 units/kg/leg, 79 patients received 15 units/kg/leg, and 77 patients received placebo. Administration was by injection into the gastrocnemius and soleus muscles. Forty one percent of patients (n=66) were treated bilaterally and received a total Dysport^{®} dose of either 20 Units/kg (n=37) or 30 Units/kg (n=29).

**Table 1**

| | Week | Placebo | 10 units/kg/leg | 15 units/kg/leg |
|---|---|---|---|---|
| LS mean change from baseline in ankle plantar flexor muscle tone on the MAS | 4 | -0.5 | -0.9* | -1.0* |
| | 12 | -0.5 | -0.8* | -1.0* |
| LS mean PGA of response to treatment | 4 | 0.7 | 1.5* | 1.5* |
| | 12 | 0.4 | 0.8* | 1.0* |
| LS = least square *p<0.05 | | | | |

The patients' change in MAS score following treatment and the PGA score for treatment were measured. As shown above, treatment with botulinum neurotoxin significantly reduced MAS in ankle plantar flexor muscle tone as compared with placebo. Treatment with botulinum neurotoxin also resulted in a significantly greater mean PGA score as compared with placebo.

### Example 2

Five hundred units of lyophilized Dysport^{®} are gently mixed with 2.5 mL of preservative-free 0.9% sodium chloride solution in a vial, forming an initial Dysport^{®} solution. An additional 2.5 mL of preservative-free 0.9% sodium chloride solution is drawn into a 5 mL syringe. The syringe is then used to draw the aforementioned Dysport^{®} solution from the vial without inverting. The resulting solution is mixed gently in the syringe to form a 10 units/0.1 mL injectable solution. The solution is stored in the syringe in a refrigerator at 2 to 8 °C and protected from light. The composition is used within 24 hours and, if not used, is discarded.

### Example 3

Three hundred units of lyophilized Dysport^{®} are gently mixed with 1.5 mL of preservative-free 0.9% sodium chloride solution in a vial, forming an initial Dysport^{®} solution. An additional 1.5 mL of preservative-free 0.9% sodium chloride solution is drawn into a 3 mL syringe. The syringe is then used to draw the aforementioned Dysport^{®} solution from the vial without inverting. The resulting solution is mixed gently in the syringe to form a 10 units/0.1 mL injectable solution. The solution is stored in the syringe in a refrigerator at 2 to 8 °C and protected from light. The composition is used within 24 hours and, if not used, is discarded.

### Example 4

A physician treating a 10-year old patient for severe lower limb spasticity in the left soleus (MAS score of 3) first obtains the weight of the patient. The weight is found to be 30 kg. As the patient is exhibiting spasticity in the soleus of his left leg, the physician determines that administration should be unilateral and to the soleus of the left leg. Consulting a dosing tool, it is determined that a dose of 120 to 180 units is appropriate. The patient's history is then reviewed. As it is found that the patient has had no adverse effects from previous treatment with botulinum neurotoxin and the level of spasticity was severe, it is determined that 180 units are to be administered.

A 10 unit/0.1 mL injectable Dysport^{®} solution in a syringe is prepared as described in Example 2. 1.8 mL of the solution is administered by injection into the patient's soleus. The syringe, along with the remainder of the solution therein, is discarded.

The above steps are repeated in 16 weeks.

### Example 5

A physician treating a 12-year old patient for minor lower limb spasticity in both left and right gastrocnemius (MAS score of 1+) first obtains the weight of the patient. The weight is found to be 40 kg. As the patient is exhibiting spasticity in the gastrocnemius of both legs, the physician determines that administration should be bilateral and to the gastrocnemius of both legs. Consulting a dosing tool, it is determined that a dose of 240 to 360 units to each leg is appropriate. The patient's history is then reviewed. As it is found that the patient has had adverse effects from previous treatment with botulinum neurotoxin and the level of spasticity was minor, it is determined that 240 units are to be administered to each leg.

A 10 unit/0.1 mL injectable Dysport^{®} solution in a syringe is prepared as described in Example 2. 2.4 mL of the solution is administered by injection into the gastrocnemius of each of the patient's legs. The syringe, along with the remainder of the solution therein, is discarded.

The above steps are repeated in 18 weeks.

### Example 6

A physician treating an 8-year old patient for minor lower limb spasticity in the left soleus and the right gastrocnemius (MAS score of 1) first obtains the weight of the patient. The weight is found to be 20 kg. The physician determines that administration should be unilaterally to the soleus of the left leg and unilaterally to the gastrocnemius of the right leg. Consulting a dosing tool, it is determined that a dose of 80 to 120 units to the left soleus and 120 to 180 units to the right gastrocnemius is appropriate. The patient's history is then reviewed. As it is found that the patient has had no adverse effects from previous treatment with botulinum neurotoxin and the level of spasticity was average, it is determined that 100 units are to be administered to the left soleus and 150 units are to be administered to the right gastrocnemius.

A 10 unit/0.1 mL injectable Dysport^{®} solution in a syringe is prepared as described in Example 2. 1 mL of the solution is administered by injection into the left soleus and 1.5 mL is administered by injection into the right gastrocnemius.

The above steps are repeated in 17 weeks.

### Example 7

A physician treating an 8-year old patient for minor lower limb spasticity in the left soleus and the right gastrocnemius (MAS score of 1) first obtains the weight of the patient. The weight is found to be 20 kg. The physician determines that administration should be unilaterally to the soleus of the left leg and unilaterally to the gastrocnemius of the right leg. Consulting a dosing tool, it is determined that a dose of 80 to 120 units to the left soleus and 120 to 180 units to the right gastrocnemius is appropriate. The patient's history is then reviewed. The patient exhibited local muscle weakness in the right gastrocnemius. However, the patient has had no adverse effects from previous treatment with botulinum neurotoxin and the level of spasticity was average. It is determined that 100 units are to be administered to the left soleus and, due to muscle weakness, 120 units are to be administered to the right gastrocnemius.

A 10 unit/0.1 mL injectable Dysport^{®} solution in a syringe is prepared as described in Example 2. 1 mL of the solution is administered by injection into the left soleus and 1.2 mL is administered by injection into the right gastrocnemius.

The above steps are repeated in 17 weeks.

### Example 8

A physician treating a 12-year old patient for minor lower limb spasticity in both left and right gastrocnemius (MAS score of 1+) first obtains the weight of the patient. The weight is found to be 40 kg. As the patient is exhibiting spasticity in the gastrocnemius of both legs, the physician determines that administration should be bilateral and to the gastrocnemius of both legs. Consulting a dosing tool, it is determined that a dose of 240 to 360 units to each leg is appropriate. The patient's history is then reviewed. It is found that the patient has had no adverse effects from previous treatment with botulinum neurotoxin but that such treatment did not achieve a desired result (PGA was 0). In view of this and that severity of the spasticity was average, it is determined that 360 units are to be administered to each leg.

A 10 unit/0.1 mL injectable Dysport^{®} solution in a syringe is prepared as described in Example 2. 3.6 mL of the solution is administered by injection into the gastrocnemius of each of the patient's legs. The syringe, along with the remainder of the solution therein, is discarded.

The above steps are repeated in 17 weeks.

### Example 9

A physician treating a 12-year old patient for minor lower limb spasticity in both left and right gastrocnemius (MAS score of 1) first obtains the weight of the patient. The weight is found to be 40 kg. As the patient is exhibiting spasticity in the gastrocnemius of both legs, the physician determines that administration should be bilateral and to the gastrocnemius of both legs. Consulting a dosing tool, it is determined that a dose of 240 to 360 units to each leg is appropriate. The patient's history is then reviewed. It is found that the patient has had no adverse effects from previous treatment with botulinum neurotoxin but that such treatment did not achieve a desired result (PGA was 0.03). In view of this and that severity of the spasticity was average, it is determined that 360 units are to be administered to each leg.

A 10 unit/0.1 mL injectable Dysport^{®} solution in a syringe is prepared as described in Example 2. 3.6 mL of the solution is administered by injection into the gastrocnemius of each of the patient's legs. The syringe, along with the remainder of the solution therein, is discarded.

The above steps are repeated in 17 weeks.

## Claims

1. A tool for use in determining a dosage amount of a botulinum neurotoxin to be administered to a subject, the tool comprising:
a first member having thereon a first row of numbers indicative of the weight of the subject and a second row of numbers indicative of a dosage amount of the botulinum neurotoxin to be administered to the subject, wherein the numbers in the first row indicating a specific weight are in latitudinal or radial alignment with corresponding numbers in the second row indicating the specific dosage amount to be administered to a subject having that specific weight, wherein the dosage amount corresponds to 5 to 15 units per kilogram of the total body weight of the subject per leg; and
a second member;
wherein the first and second members are in a relationship with one another such that one member may be moved by a user to provide an indication as to the dosage amount of the botulinum neurotoxin to be administered to a subject having a particular weight; and
wherein the first member further has thereon a row of numbers indicative of the dosage amount of the botulinum neurotoxin to be administered to a particular muscle of the subject and the numbers in the row which indicate the specific dosage amount to be administered to the muscle of a subject having a specific weight are in longitudinal or radial alignment with corresponding numbers in the first row indicating that specific weight, wherein the dosage amount corresponds to 6 to 9 units per kilogram of the total body weight of the subject to be administered to an individual gastrocnemius muscle of the subject or wherein the dosage amount corresponds to 4 to 6 units per kilogram of the total body weight of the subject to be administered to an individual soleus muscle of the subject.

2. The tool of claim 1, wherein the first and second members are both planar surfaces that are in a longitudinal sliding arrangement with each another, the numbers in the first row indicating a specific weight are in latitudinal alignment with corresponding numbers in the second row indicating a specific dosage amount to be administered to a subject having that specific weight, and the second member overlays the first and contains an opening that allows for the rows on the first member to be viewed or which comprises two or more openings with each opening allowing for one row on the first member to be viewed, wherein one member may be slid in relation to the other with the numbers viewable through the opening or openings indicating a specific weight and the dosage amount to be administered to a subject having that specific weight.

3. The tool of claim 1 or 2, wherein:
(i) the first member is a planar surface and the second member is a cursor in a longitudinal sliding arrangement with respect to the first member, the numbers in the first row indicating a specific weight are in latitudinal alignment with corresponding numbers in the second row indicating a specific dosage amount to be administered to a subject having that specific weight, and the cursor may be slid along the first member such that it indicates a specific weight and the dosage amount to be administered to a subject having that specific weight; or
(ii) the first member is a circular planar surface and the second member is a dial that pivots around the center point of the first member, the numbers in the first row indicating a specific weight are in radial alignment with corresponding numbers in the second row indicating a specific dosage amount to be administered to a subject having that specific weight, wherein the cursor may be pivoted such that it indicates a specific weight and the dosage amount to be administered to a subject having that specific weight.

4. The tool of claim 3, wherein:
(i) the indication is by way of a line on the cursor which overlays a specific weight and the dosage amount to be administered to a subject having that specific weight; or
(ii) the indication is by way of an opening in the cursor that allows for the rows on the first member to be viewed or two or more openings with each opening allowing for one row on the first member to be viewed and wherein the numbers viewable through the opening or openings indicate a specific weight and the dosage amount to be administered to a subject having that specific weight.

5. The tool of any one of the preceding claims, wherein:
(i) the first member further has thereon two rows of numbers, each row indicative of the dosage amount of the botulinum neurotoxin to be administered to different muscles of the subject and the numbers in the rows which indicate a specific dosage amount to be administered to the muscles of a subject having a specific weight are in longitudinal or radial alignment with corresponding numbers in the first row indicating that specific weight; or
(ii) the second row indicates the total dosage amount of the botulinum neurotoxin to be administered to a single leg of the subject and the first member further has thereon two further rows of numbers, one row indicating the dosage amount of the botulinum neurotoxin to be administered to the gastrocnemius of the leg and another row indicating the dosage amount of the botulinum neurotoxin to be administered to the soleus of the leg, the numbers in the rows which indicate a specific dosage amount to be administered to the muscles of a subject having a specific weight being in longitudinal or radial alignment with corresponding numbers in the first row indicating that specific weight.

## Patentansprüche

1. Vorrichtung zur Verwendung beim Bestimmen einer einem Subjekt zu verabreichenden Dosierungsmenge eines Botulinum-Neurotoxins, wobei die Vorrichtung Folgendes umfasst:
ein erstes Element, das eine erste Reihe von Zahlen, die das Gewicht des Subjekts angeben, und eine zweite Reihe von Zahlen aufweist, die eine dem Subjekt zu verabreichende Dosierungsmenge des Botulinum-Neurotoxins angeben, wobei die Zahlen in der ersten Reihe, die ein spezifisches Gewicht angeben, latitudinal oder radial mit entsprechenden Zahlen in der zweiten Reihe fluchten, die die einem Subjekt mit diesem spezifischen Gewicht zu verabreichende spezifische Dosierungsmenge angeben, wobei die Dosierungsmenge 5 bis 15 Einheiten pro Kilogramm des Gesamtkörpergewichts des Subjekts pro Bein entspricht; und
ein zweites Element;
wobei das erste und das zweite Element in einer solchen Beziehung zueinander stehen, dass ein Element von einem Benutzer bewegt werden kann, um eine Anzeige der einem Subjekt mit einem spezifischen Gewicht zu verabreichenden Dosierungsmenge des Botulinum-Neurotoxins zu erhalten; und
wobei das erste Element ferner eine Reihe von Zahlen aufweist, die die einem bestimmten Muskel des Subjekts zu verabreichende Dosierungsmenge des Botulinum-Neurotoxins angeben, und die Zahlen in der Reihe, die die dem Muskel eines Subjekts mit einem spezifischen Gewicht zu verabreichende spezifische Dosierungsmenge angeben, longitudinal oder radial mit entsprechenden Zahlen in der ersten Reihe fluchten, die dieses spezifische Gewicht angeben, wobei die Dosierungsmenge 6 bis 9 Einheiten pro Kilogramm des Gesamtkörpergewichts des Subjekts entspricht, die einem einzelnen Zwillingswadenmuskel des Subjekts zu verabreichen sind, oder wobei die Dosierungsmenge 4 bis 6 Einheiten pro Kilogramm des Gesamtkörpergewichts des Subjekts entspricht, die einem einzelnen Schollenmuskel des Subjekts zu verabreichen sind.

2. Vorrichtung nach Anspruch 1, wobei das erste und das zweite Element beide ebene Flächen sind, die longitudinal gleitend zueinander angeordnet sind, wobei die Zahlen in der ersten Reihe, die ein spezifisches Gewicht angeben, latitudinal mit entsprechenden Zahlen in der zweiten Reihe fluchten, die eine einem Subjekt mit diesem spezifischen Gewicht zu verabreichende spezifische Dosierungsmenge angeben, und das zweite Element das erste überlagert und eine Öffnung hat, die eine Betrachtung der Reihen auf dem ersten Element ermöglicht, oder zwei oder mehr Öffnungen hat, wobei jede Öffnung eine Betrachtung einer Reihe auf dem ersten Element ermöglicht, wobei ein Element in Bezug auf das andere verschoben werden kann, wobei die durch die Öffnung(en) sichtbaren Zahlen ein spezifisches Gewicht und die einem Subjekt mit diesem spezifischen Gewicht zu verabreichende Dosierungsmenge angeben.

3. Vorrichtung nach Anspruch 1 oder 2, wobei:
(i) das erste Element eine ebene Fläche ist und das zweite Element ein Positionsanzeiger ist, der in Bezug auf das erste Element longitudinal gleitend angeordnet ist, wobei die Zahlen in der ersten Reihe, die ein spezifisches Gewicht angeben, latitudinal mit entsprechenden Zahlen in der zweiten Reihe fluchten, die eine einem Subjekt mit diesem spezifischen Gewicht zu verabreichende spezifische Dosierungsmenge angeben, und der Positionsanzeiger entlang des ersten Elements verschoben werden kann, so dass er ein spezifisches Gewicht und die einem Subjekt mit diesem spezifischen Gewicht zu verabreichende Dosierungsmenge anzeigt; oder
(ii) das erste Element eine kreisförmige ebene Fläche ist und das zweite Element eine Anzeigeskala ist, die sich um den Mittelpunkt des ersten Elements dreht, wobei die Zahlen in der ersten Reihe, die ein spezifisches Gewicht angeben, radial mit den entsprechenden Zahlen in der zweiten Reihe fluchten, die eine einem Subjekt mit diesem spezifischen Gewicht zu verabreichende spezifische Dosierungsmenge angeben, wobei der Positionsanzeiger so gedreht werden kann, dass er ein spezifisches Gewicht und die einem Subjekt mit diesem spezifischen Gewicht zu verabreichende Dosierungsmenge anzeigt.

4. Vorrichtung nach Anspruch 3, wobei:
(i) die Anzeige durch eine Linie auf dem Positionsanzeiger erfolgt, die über einem spezifischen Gewicht und der einem Subjekt mit diesem spezifischen Gewicht zu verabreichenden Dosierungsmenge liegt; oder
(ii) die Anzeige durch eine Öffnung im Positionsanzeiger erfolgt, die eine Betrachtung der Reihen auf dem ersten Element ermöglicht, oder durch zwei oder mehr Öffnungen, wobei jede Öffnung die Betrachtung einer Reihe auf dem ersten Element ermöglicht, und wobei die durch die Öffnung(en) sichtbaren Zahlen ein spezifisches Gewicht und die einem Subjekt mit diesem spezifischen Gewicht zu verabreichende Dosierungsmenge angeben.

5. Vorrichtung nach einem der vorherigen Ansprüche, wobei:
(i) das erste Element ferner zwei Reihen von Zahlen aufweist, wobei die jeweiligen Reihen die verschiedenen Muskeln des Subjekts zu verabreichende Dosierungsmenge des Botulinum-Neurotoxins angeben, und die Zahlen in den Reihen, die eine den Muskeln eines Subjekts mit einem spezifischen Gewicht zu verabreichende spezifische Dosierungsmenge angeben, longitudinal oder radial mit entsprechenden Zahlen in der ersten Reihe fluchten, die dieses spezifische Gewicht angeben; oder
(ii) die zweite Reihe die einem einzelnen Bein des Subjekts zu verabreichende Gesamtdosierungsmenge des Botulinum-Neurotoxins angibt, und das erste Element ferner zwei weitere Zahlenreihen aufweist, wobei eine Reihe die dem Zwillingswadenmuskel des Beins zu verabreichende Dosierungsmenge des Botulinum-Neurotoxins angibt, und eine andere Reihe die dem Schollenmuskel des Beins zu verabreichende Dosierungsmenge des Botulinum-Neurotoxins angibt, wobei die Zahlen in den Reihen, die eine den Muskeln eines Subjekts mit einem spezifischen Gewicht zu verabreichende spezifische Dosierungsmenge angeben, longitudinal oder radial mit entsprechenden Zahlen in der ersten Reihe, die dieses spezifische Gewicht angeben, fluchten.

## Revendications

1. Outil destiné à une utilisation pour déterminer un dosage d'une neurotoxine botulique à administrer à un sujet, l'outil comprenant :
un premier élément portant une première rangée de nombres indiquant le poids du sujet et une deuxième rangée de nombres indiquant un dosage de la neurotoxine botuline à administrer au sujet, les nombres dans la première rangée indiquant un poids spécifique étant alignés latitudinalement ou radialement avec des nombres correspondants dans la deuxième rangée indiquant le dosage spécifique à administrer à un sujet ayant ce poids spécifique, le dosage correspondant à 5 à 15 unités par kilogramme du poids corporel total du sujet par jambe ; et
un deuxième élément ;
dans lequel les premier et deuxième éléments sont dans une situation l'un par rapport à l'autre telle qu'un élément puisse être déplacé par un utilisateur pour fournir une indication quant au dosage de la neurotoxine botulique à administrer à un sujet ayant un poids particulier ; et
dans lequel le premier élément porte en outre une rangée de nombres indiquant le dosage de la neurotoxine botulique à administrer dans un muscle particulier du sujet et les nombres dans la rangée qui indiquent le dosage spécifique à administrer dans le muscle d'un sujet ayant un poids spécifique étant alignés longitudinalement ou radialement avec des nombres correspondants dans la première rangée indiquant ce poids spécifique, le dosage correspondant à 6 à 9 unités par kilogramme du poids corporel total du sujet à administrer dans un muscle gastrocnémien individuel du sujet ou le dosage correspondant à 4 à 6 unités par kilogramme du poids corporel total du sujet à administrer dans un muscle soléaire du sujet.

2. Outil selon la revendication 1, dans lequel les premier et deuxième éléments sont tous deux des surfaces planes qui sont disposées de façon à coulisser longitudinalement l'une par rapport à l'autre, les nombres dans la première rangée indiquant un poids spécifique étant alignés latitudinalement avec des nombres correspondants dans la deuxième rangée indiquant un dosage spécifique à administrer à un sujet ayant ce poids spécifique, et le deuxième élément se superposant sur le premier et contenant une ouverture qui permet de visualiser les rangées sur le premier élément ou comprenant deux ouvertures ou plus, chaque ouverture permettant de visualiser une rangée sur le premier élément, un élément pouvant être coulissé par rapport à l'autre, les nombres visibles à travers l'ouverture ou les ouvertures indiquant un poids spécifique et le dosage à administrer à un sujet ayant ce poids spécifique.

3. Outil selon la revendication 1 ou 2, dans lequel :
(i) le premier élément est une surface plane et le deuxième élément est un curseur disposé de façon à coulisser longitudinalement par rapport au premier élément, les nombres dans la première rangée indiquant un poids spécifique étant alignés latitudinalement avec des nombres correspondants dans la deuxième rangée indiquant un dosage spécifique à administrer à un sujet ayant ce poids spécifique et le curseur pouvant être coulissé le long du premier élément de façon à indiquer un poids spécifique et le dosage à administrer à un sujet ayant ce poids spécifique ; ou
(ii) le premier élément est une surface plane circulaire et le deuxième élément est un cadran qui pivote autour du point central du premier élément, les nombres dans la première rangée indiquant un poids spécifique étant alignés radialement avec des nombres correspondants dans la deuxième rangée indiquant un dosage spécifique à administrer à un sujet ayant ce poids spécifique, le curseur pouvant être pivoté de façon à indiquer un poids spécifique et le dosage à administrer à un sujet ayant ce poids spécifique.

4. Outil selon la revendication 3, dans lequel :
(i) l'indication se fait au moyen d'une ligne sur le curseur qui se superpose sur un poids spécifique et le dosage à administrer à un sujet ayant ce poids spécifique ; ou
(ii) l'indication se fait au moyen d'une ouverture dans le curseur qui permet de visualiser les rangées sur le premier élément ou de deux ouvertures ou plus, chaque ouverture permettant de visualiser une rangée sur le premier élément et les nombres visibles à travers l'ouverture ou les ouvertures indiquant un poids spécifique et le dosage à administrer à un sujet ayant ce poids spécifique.

5. Outil selon l'une quelconque des revendications précédentes, dans lequel :
(i) le premier élément porte en outre deux rangées de nombres, chaque rangée indiquant le dosage de la neurotoxine botulique à administrer dans différents muscles du sujet et les nombres dans les rangées qui indiquent un dosage spécifique à administrer dans les muscles d'un sujet ayant un poids spécifique étant alignés longitudinalement ou radialement avec des nombres correspondants dans la première rangée indiquant ce poids spécifique ; ou
(ii) la deuxième rangée indique le dosage total de la neurotoxine botulique à administrer dans une seule jambe du sujet et le premier élément porte en outre deux autres rangées de nombres, une rangée indiquant le dosage de la neurotoxine botulique à administrer dans le gastrocnémien de la jambe et une autre rangée indiquant le dosage de la neurotoxine botulique à administrer dans le soléaire de la jambe, les nombres dans les rangées qui indiquent un dosage spécifique à administrer dans les muscles d'un sujet ayant un poids spécifique étant alignés longitudinalement ou radialement avec des nombres correspondants dans la première rangée indiquant ce poids spécifique.
